# EUROPEAN PATENT APPLICATION

(11) **EP 4 474 386 A1**
(43) Date of publication of application: **11.12.2024**
(21) Application number: 23177913.3
(22) Date of filing: 07.06.2023
(51) Int. Cl.: C07H 15/252, A61K 31/704, A61P 35/02

(54) **ANTHRACYCLINES**

(71) Applicant: Academisch Ziekenhuis Leiden h.o.d.n. LUMC, 2333 ZA Leiden (NL); Universiteit Leiden, 2311 RA Leiden (NL)
(72) Inventor: NEEFJES, Jacques J.C., 2333 CZ Leiden (NL); OVERKLEEFT, Herman S., 2333 CC (NL); WANDER, Dennis P.A., 2333 CC (NL); VAN DER ZANDEN, Sabina Y., 2333 CZ Leiden (NL); VAN GELDER, Merle A., 2333 CZ Leiden (NL)
(74) Representative: HGF

(57) **Abstract**

Provided herein are compounds useful in the treatment of cancer, as well as formulations and compositions comprising such compounds. Also provided is the use of such compounds, formulations and compositions as a medicament, for example in the treatment of cancer. The compounds are of formula I: 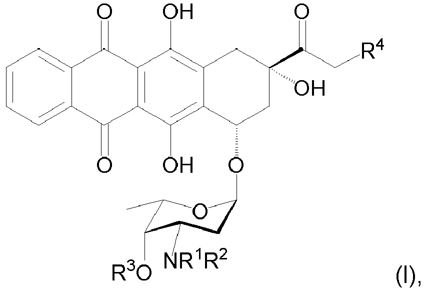 or a pharmaceutically acceptable salt, stereoisomer, solvate, or prodrug thereof. R¹ is a substituted or unsubstituted C₁₋₄ alkyl. R² is a substituted or unsubstituted C₁₋₄ alkyl. R³ is selected from H, monosaccharide, and disaccharide. R⁴ is selected from H, and OH.

## Description

This invention relates to novel anthracycline compounds useful in the treatment of cancer, as well as formulations and compositions comprising such compounds. Also provided is the use of such compounds, formulations and compositions as a medicament, for example in the treatment of cancer.

### BACKGROUND

Anthracyclines are extensively used as chemotherapeutics in the treatment of various haematological cancers and solid tumors since their discovery in the 1960s. Because of their broad anti-cancer effectivity, they are considered 'essential medicines' by the WHO, and their remarkable potency has inspired the development of thousands of variants. Only a few of these analogues have been approved for clinical use, of which only doxorubicin, daunorubicin, epirubicin and idarubicin have been adopted for worldwide use.

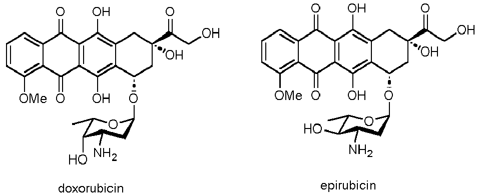

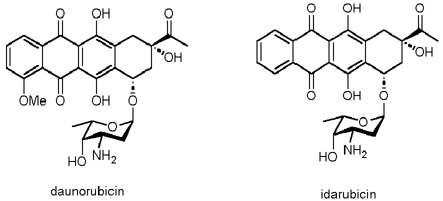

While these anthracyclines are among the most effective anti-cancer drugs, their clinical application is hampered by treatment-limiting side effects and drug resistance. The side effects of anthracycline treatment are severe: cardiotoxicity, secondary tumor formation and infertility affect the quality of life and survival of patients, regardless of the cancer prognosis. Of these, cardiotoxicity is the main adverse effect which emerges in a cumulative manner and is restricting treatment regimens as a consequence.

It is an object of the present invention to provide anthracycline variant compounds with improved anti-cancer activity compared to that of doxorubicin.

It is another object of the present invention to provide anthracycline variant compounds that may be better tolerated by patients compared to that of doxorubicin.

It is another object of the present invention to provide anthracycline variant compounds that show retained and/or enhanced cytotoxic potency in resistant cancer cells compared to that shown by doxorubicin.

### BRIEF SUMMARY OF THE DISCLOSURE

It has been found that the compounds of the present invention show activity that renders them useful in the prophylaxis or treatment of cancer in homo sapiens. In particular, the present compounds exhibit beneficial properties which indicate their ability to treat cancer in patients whilst also indicating they could show a reduction in the severity of the long-term side effects typically associated with the anthracycline drugs that are currently in clinical use. Notably, compounds of the present invention can show cytotoxicity comparable to or better than that of doxorubicin and idarubicin, whilst also displaying comparable or improved chromatin damage, but without inducing DNA double-strand breaks.

The present compounds thus have the potential to provide effective anthracycline chemotherapeutics, with reduced treatment-limiting side effects. The compounds may demonstrate reduced cancer drug resistance (e.g. compared to doxorubicin and/or idarubicin). The compounds may provide reduced off-target side effects, such as relatively low cardiotoxicity. Without wishing to be bound by any theory, it is believed that the present compounds could provide such benefits by inducing requisite chromatin damage without also inducing double stranded DNA breaks.

A first aspect of the invention provides a compound of formula I, or a pharmaceutically acceptable salt, stereoisomer, solvate or prodrug thereof: 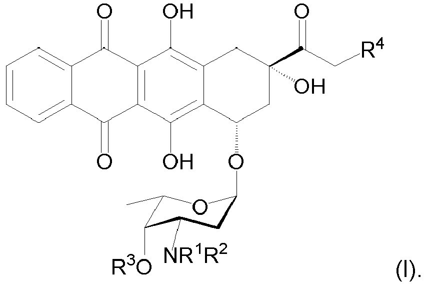 R¹ is a substituted or unsubstituted C₁₋₄ alkyl. R² is a substituted or unsubstituted C₁₋₄ alkyl. R³ is selected from H, monosaccharide, and disaccharide. R⁴ is selected from H, and OH.

In an embodiment, R¹ is an unsubstituted C₁₋₄ alkyl. R¹ may be selected from CH₃, CH₂CH₃, CH(CH₃)₂ and CH₂CH₂CH₃. R¹ may be selected from CH₃ and CH₂CH₃. R¹ may be CH₃. R¹ may be CH₂CH₃.

In an embodiment, R² is an unsubstituted C₁₋₄ alkyl. R² may be selected from CH₃, CH₂CH₃, CH(CH₃)₂ and CH₂CH₂CH₃. R² may be selected from CH₃ and CH₂CH₃. R² may be CH₃. R² may be CH₂CH₃.

In an embodiment, R¹ is CH₃ and R² is CH₃.

In an embodiment, R¹ and / or R² is a substituted C₁₋₄ alkyl. The or each substituted C₁₋₄ alkyl may be of formula II: 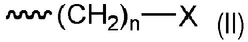 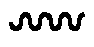 designates the point of attachment to the rest of the compound. n is 1, 2, 3 or 4, e.g. n is 2 or 3. X is OH, halogen, or -OSO₂R⁵. R⁵ is a C₁₋₄ alkyl or aryl optionally substituted by C₁₋₄ alkyl, nitro, amino, methoxy, or halogen. For example, n may be 2 or 3, and X may be OH or halogen. Where both R¹ and R² are of formula II, each moiety of formula II may be the same, or each moiety of formula II may be different.

In an embodiment, R³ is selected from H, and where 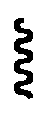 designates the point of attachment to the rest of the compound. R³ may be H. R³ may be

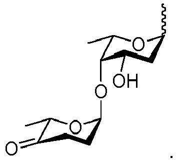

In an embodiment, R⁴ is H. In another embodiment, R⁴ is OH.

In an embodiment, the compound of the invention is selected from: 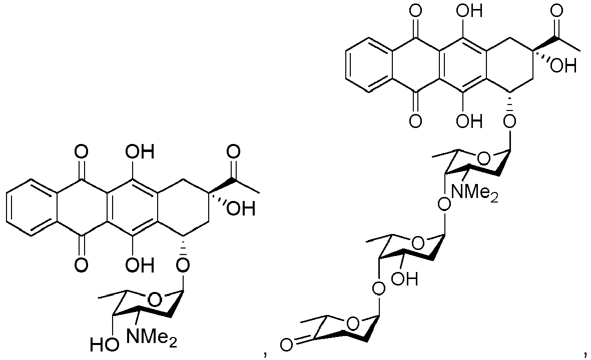 or a pharmaceutically acceptable salt, stereoisomer, solvate, or prodrug thereof.

In an embodiment the compound has an IC₅₀ of less than about 300 nM against cell line K562 and wherein the fraction of broken DNA damage in the K562 cells is less than about 0.10. In an embodiment the compound has an IC₅₀ of less than about 200 nM against cell line K562 and wherein the fraction of broken DNA damage in the K562 cells is less than about 0.10. For example, the IC₅₀ may be less than about 150 nM against cell line K562, e.g. the IC₅₀ may be less than about 125 nM. Preferably, the IC₅₀ may be less than about 100 nM against cell line K562, e.g. the IC₅₀ may be not more than about 80 nM. For example, the fraction of broken DNA damage in the K562 cells may be less than about 0.05.

In an embodiment the compound has an IC₅₀ of less than about 200 nM against cell line MelJuSo and/or an IC₅₀ of less than about 200 nM against cell line U2Os. For example, the IC₅₀ may be less than about 150 nM against cell line MelJuSo and/or the IC₅₀ may be less than about 150 nM against cell line U2Os; e.g. the IC₅₀ may be less than about 125 nM against cell line MelJuSo and/or the IC₅₀ may be less than about 125 nM against cell line U2Os.

A second aspect of the invention provides a formulation or composition comprising at least one compound of the first aspect and optionally a pharmaceutically acceptable carrier. The formulation or composition is typically a pharmaceutical formulation or pharmaceutical composition.

In an embodiment, the formulation or composition is an oral formulation. For example, the formulation or composition may be formulated for oral administration.

A third aspect of the invention provides a compound of the first aspect, or formulation or composition of the second aspect, for use as a medicament.

A fourth aspect of the invention provides a compound of the first aspect or a formulation or composition of the second aspect for use in the treatment of cancer.

In an embodiment, the cancer is selected from a haematological cancer or a solid tumor. The haematological cancer may be a leukemia, such as acute myeloid leukemia (AML) or acute lymphatic leukemia (ALL). The solid tumor may be a breast cancer.

The cancer may be a resistant cancer. For example, the cancer may be resistant to treatment by doxorubicin. In an embodiment, the cancer may comprise an ATP binding cassette B1 (ABCB1) OE chemotherapy resistance mechanism.

### BRIEF DESCRIPTION OF THE DRAWINGS

Embodiments of the invention are further described hereinafter with reference to the accompanying drawings, in which:

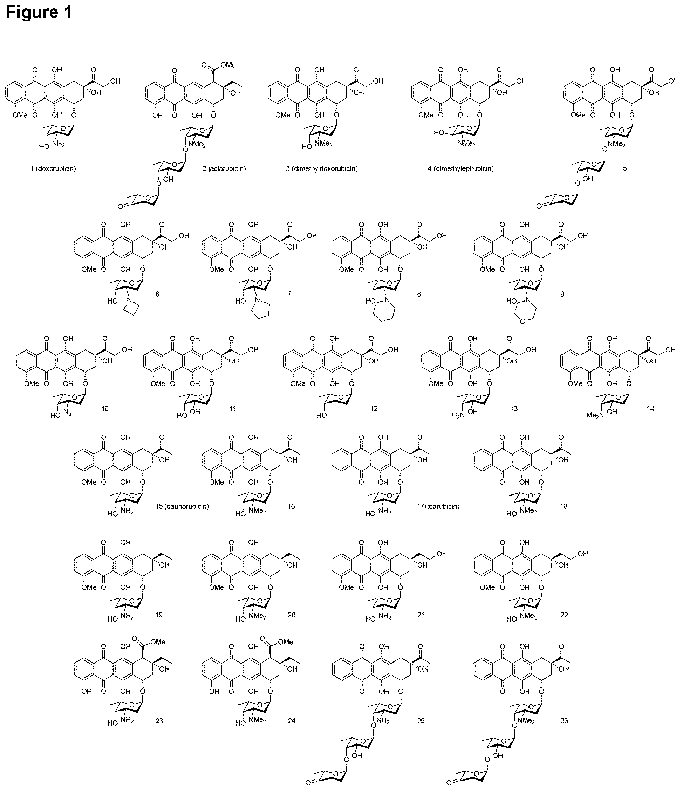

**Figure 1** shows the structures of 26 anthracycline derivatives that were evaluated, including compounds of the invention and additional compounds. These contain the clinically used anthracyclines doxorubicin (**1**), aclarubicin (**2**), daunorubicin (**15**), and idarubicin (**17**).

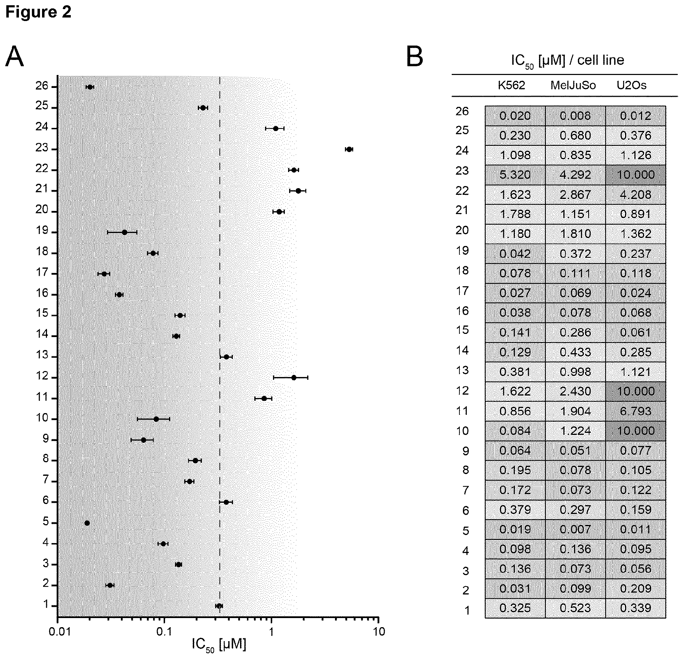

**Figure 2** shows the anti-cancer potency of anthracycline derivatives on three different tumor cell lines. Numbers correspond to the structures in FIG. 1. (A) IC₅₀ values are plotted for all derivatives tested in the human myelogenous leukemic cell line K562. The dotted line indicates the IC₅₀ of doxorubicin (1). The Y axis shows the number of the structure shown in FIG. 1. (B) IC₅₀ values for the 26 anthracycline variants tested in human myelogenous leukemic cell line K562, human melanoma cell line MelJuSo and human osteosarcoma cell line U2OS.

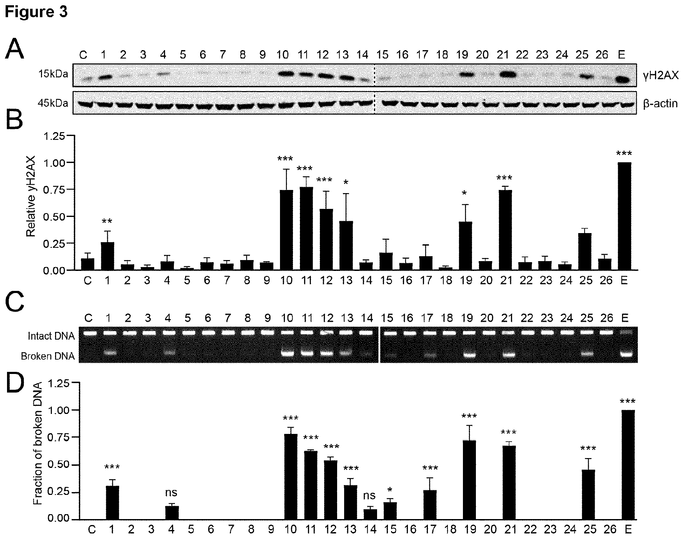

**Figure 3** shows the DNA damage capacity of the full set of anthracycline derivatives. Numbers correspond to the structures in FIG. 1. (A) K562 cells were treated for 2 h with 10 µM of the indicated compounds, etoposide [E] was used as positive control. γH2AX levels were examined by Western blot. Actin was used as a loading control, and molecular weight markers are indicated. (B) Quantification of γH2AX signal normalized to the loading control. Results are presented as mean ± SD of three independent experiments. Ordinary one-way ANOVA with Dunnett's multiple comparison test. *P < 0.05, **P < 0.01, ***P < 0.001. (C) DNA double-strand breaks were directly visualized by CFGE. The position of intact and broken DNA is indicated. (D) Quantification of broken DNA relative to total DNA as analysed by CFGE. Etoposide [E] was used as positive control. Results are presented as mean ± SD of three independent experiments. Ordinary one-way ANOVA with Dunnett's multiple comparison test. *P < 0.05, ***P < 0.001.

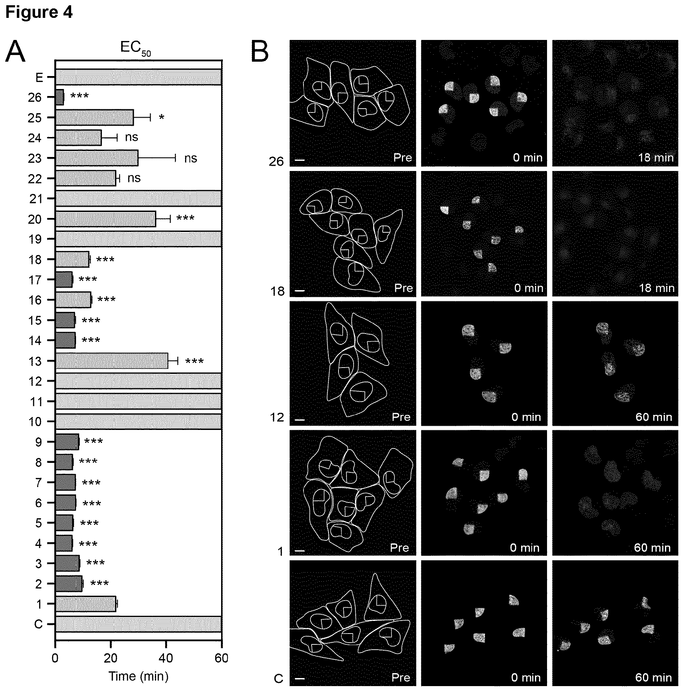

**Figure 4** shows the chromatin damage efficacy of the anthracycline derivatives. (A) The rate of histone eviction for all derivatives is plotted as EC₅₀ (time at which 50% of the initial signal in the photoactivated spot is reduced). Etoposide [E] was used as negative control. Nonlinear regression with sum-of-squares F test. *P < 0.05, ***P < 0.001, ns = not significant. (B) Illustration of the effects of indicated compounds (numbers on left side indicate the drug in FIG. 1) on eviction of the photoactivated histones. Left panel: drawn cell out line and nucleus with the photoactivated part of the nucleus in living MelJuSo-PAGFP-H2A cells. Middle panel shows the photoactivated histones at the onset of the experiment after compound addition. Photo-activation was monitored by time-lapse confocal microscopy for 1 hour in the presence of the indicated drugs at 10 µM. Stills made at 60 min are shown in the right panel. Scale bar, 10 µm.

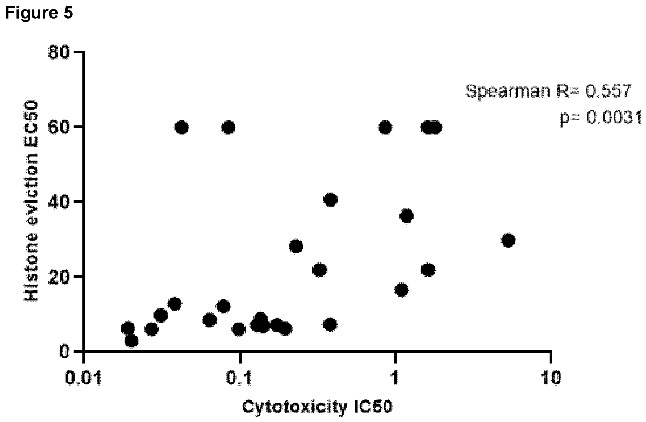

**Figure 5** shows a graphical representation of the relationship between cytotoxicity (IC₅₀) and rate of histone eviction (EC₅₀).

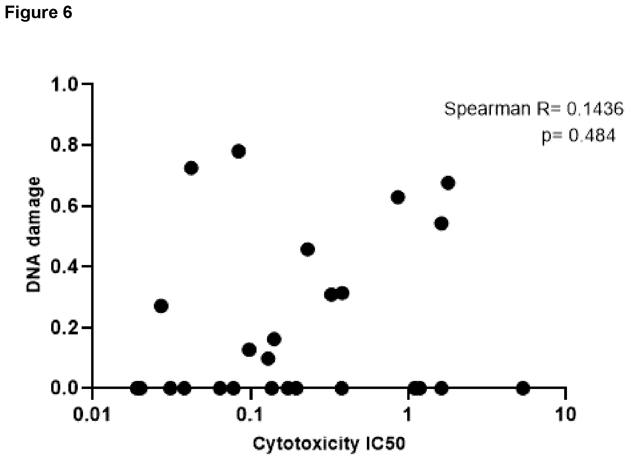

**Figure 6** shows a graphical representation of the relationship between cytotoxicity (IC₅₀) and DNA damaging capacity (expressed as a fraction of broken DNA).

### DETAILED DESCRIPTION

The abbreviations used herein have their conventional meaning within the chemical and biological arts.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The reader's attention is directed to all papers and documents which are filed concurrently with or previous to this specification in connection with this application and which are open to public inspection with this specification, and the contents of all such papers and documents are incorporated herein by reference.

For the avoidance of doubt, it is hereby stated that the information disclosed earlier in this specification under the heading "Background" is relevant to the invention and is to be read as part of the disclosure of the invention.

All publications, patent applications, patents, and other references mentioned herein are incorporated by reference in their entirety. In case of conflict, the present specification, including definitions, will control.

### DEFINITIONS

The following explanations of terms and methods are provided to better describe the present disclosure and to guide those of ordinary skill in the art in the practice of the present disclosure.

The invention concerns amongst other things the treatment of a disease. The terms "treat", "treating", or "treatment", and the therapies encompassed by this invention, include the following and combinations thereof: (1) hindering, e.g. delaying initiation and/or progression of, an event, state, disorder or condition, for example arresting, reducing or delaying the development of the event, state, disorder or condition, or a relapse thereof in case of maintenance treatment or secondary prophylaxis, or of at least one clinical or subclinical symptom thereof; (2) preventing or delaying the appearance of clinical symptoms of an event, state, disorder or condition developing in an animal (e.g. human) that may be afflicted with or predisposed to the state, disorder or condition but does not yet experience or display clinical or subclinical symptoms of the state, disorder or condition; and/or (3) relieving and/or curing an event, state, disorder or condition (e.g., causing regression of the event, state, disorder or condition or at least one of its clinical or subclinical symptoms, curing a patient or putting a patient into remission). The benefit to a patient to be treated may be either statistically significant or at least perceptible to the patient or to the physician. It will be understood that a medicament will not necessarily produce a clinical effect in each patient to whom it is administered; thus, in any individual patient or even in a particular patient population, a treatment may fail or be successful only in part, and the meanings of the terms "treatment", "prophylaxis" and "inhibitor" and of cognate terms are to be understood accordingly. The compositions and methods described herein are of use for therapy and/or prophylaxis of the mentioned conditions.

The term "prophylaxis" includes reference to treatment therapies for the purpose of preserving health or inhibiting or delaying the initiation and/or progression of an event, state, disorder or condition, for example for the purpose of reducing the chance of an event, state, disorder or condition occurring. The outcome of the prophylaxis may be, for example, preservation of health or delaying the initiation and/or progression of an event, state, disorder or condition. It will be recalled that, in any individual patient or even in a particular patient population, a treatment may fail, and this paragraph is to be understood accordingly.

The term "inhibit" (and "inhibiting") includes reference to delaying, stopping, reducing the incidence of, reducing the risk of and/or reducing the severity of an event, state, disorder or condition. Inhibiting an event, state, disorder or condition may therefore include delaying or stopping initiation and/or progression of such, and reducing the risk of such occurring. The products of the disclosure, such as compounds, formulation or compositions of the invention, may be used to inhibit solid and haematological tumors.

The term "alkyl" as used herein include reference to a straight or branched chain alkyl moiety having 1, 2, 3, 4, 5, 6, 7, 8, 9 or 10 carbon atoms. The term includes reference to, for example, methyl, ethyl, propyl (n-propyl or isopropyl), butyl (n-butyl, sec-butyl or tert-butyl), and the like. In particular, alkyl may be a "C₁-C₄ alkyl", i.e. an alkyl having 1, 2, 3 or 4 carbon atoms; or a "C₁-C₆ alkyl", i.e. an alkyl having 1, 2, 3, 4, 5 or 6 carbon atoms; or a "C₁-C₃ alkyl", i.e. an alkyl having 1, 2 or 3 carbon atoms. A substituted or unsubstituted C₁-C₄ alkyl group may be, but is not limited to, substituted or unsubstituted methyl, substituted or unsubstituted ethyl, substituted or unsubstituted propyl ((n-propyl or isopropyl) and substituted or unsubstituted butyl butyl (n-butyl, sec-butyl or tert-butyl) group.

The term "aryl" as used herein means an aromatic group containing, suitably, 5 to 14 ring atoms. For example aryl may be phenyl or naphthyl, e.g. aryl may be phenyl. The aromatic group may be a heteroaromatic group containing one, two, three or four, preferably one, heteroatoms selected, independently, from the group consisting of O, N and S. Examples of such heteroaromatic groups include pyridyl, pyrrolyl, furanyl and thiophenyl.

The terms "halo" or "halogen" as used herein includes reference to F, Cl, Br or I, for example F, Cl or Br. In a particular class of embodiments, halogen is F or CI, of which F is more common.

The term "monosaccharide" means a carbohydrate that cannot be broken down into simpler sugars by hydrolysis. In the context of the present disclosure, a monosaccharide group includes reference to a monosaccharide moiety that comprises one or more (e.g. 1 or 2) covalent linkages to other moieties. A monosaccharide may comprise 6 carbon atoms.

The term "disaccharide" group means a class of sugars made up of two monosaccharides linked by an ether formation ( '-C-O-C-' bridge) wherein the oxygen atom links the two monosaccharides. In the context of the present disclosure, a disaccharide group includes reference to a disaccharide moiety that comprises at least 1 covalent linkage to another moiety.

The term "substituted" as used herein in reference to a moiety means that one or more, especially up to 5, more especially 1, 2 or 3, of the hydrogen atoms in said moiety are replaced independently of each other by the corresponding number of the described substituents. Unless otherwise specified, exemplary substituents include -OH, -CN, -NH₂, =O, -halo, -C₁-C₆ alkyl, -C₂-C₆ alkenyl, -C₁-C₆ haloalkyl, -C₁-C₆ haloalkoxy and-C₂-C₆ haloalkenyl, -C₁-C₆ alkylcarboxylic acid (e.g. -CH₃COOH or -COOH). Where the substituent is a -C₁-C₆ alkyl or -C₁-C₆ haloalkyl, the C₁-C₆ chain is optionally interrupted by an ether linkage (-O-) or an ester linkage (-C(O)O-). Exemplary substituents for a substituted alkyl may include-OH, -CN, -NH₂, =O, -halo, -CO₂H, -C₁-C₆ haloalkyl, -C₁-C₆ haloalkoxy and-C₂-C₆haloalkenyl, -C₁-C₆ alkylcarboxylic acid (e.g. -CH₃COOH or -COOH). For example, exemplary substituents for an alkyl may include -OH, -CN, -NH₂, =O, -halo. An exemplary substituted alkyl may have formula II: 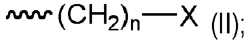 where n and X are as defined elsewhere in the present application.

It will, of course, be understood that substituents are only at positions where they are chemically possible, the person skilled in the art being able to decide (either experimentally or theoretically) without inappropriate effort whether a particular substitution is possible. For example, amino or hydroxy groups with free hydrogen may be unstable if bound to carbon atoms with unsaturated (e.g. olefinic) bonds. Additionally, it will of course be understood that the substituents described herein may themselves be substituted by any substituent, subject to the aforementioned restriction to appropriate substitutions as recognised by the skilled person.

Where steric issues determine placement of substituents on a group, the isomer having the lowest conformational energy may be preferred.

Where a compound, moiety, process or product is described as "optionally" having a feature, the disclosure includes such a compound, moiety, process or product having that feature and also such a compound, moiety, process or product not having that feature. Thus, when a moiety is described as "optionally substituted", the disclosure comprises the unsubstituted moiety and the substituted moiety.

The symbol 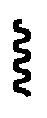 denotes a point of attachment of a moiety to the remainder of a compound.

Where two or more moieties are described as being "independently" or "each independently" selected from a list of atoms or groups, this means that the moieties may be the same or different. The identity of each moiety is therefore independent of the identities of the one or more other moieties.

The term "pharmaceutical formulation" as used herein includes reference to a formulation comprising at least one active compound and optionally one or more additional pharmaceutically acceptable ingredients, for example a pharmaceutically acceptable carrier. Where a pharmaceutical formulation comprises two or more active compounds, or comprises at least one active compound and one or more additional pharmaceutically acceptable ingredients, the pharmaceutical formulation is also a pharmaceutical composition. Unless the context indicates otherwise, all references to a "formulation" herein are references to a pharmaceutical formulation.

The term "product" or "product of the invention" as used herein includes reference to any product containing a compound of the present invention. In particular, the term product relates to compositions and formulations containing a compound of the present invention, such as a pharmaceutical composition, for example.

The term "effective amount" refers to an amount sufficient to attain the desired result. The term "therapeutically effective amount" as used herein refers to an amount of a drug, or pharmaceutical agent that, within the scope of sound pharmacological judgment, is calculated to (or will) provide a desired therapeutic response in a mammal (animal or human). The therapeutic response may for example serve to cure, delay the progression of or prevent a disease, disorder or condition. Pharmaceutical formulations and compositions provided herein preferably comprise a therapeutically effective amount of an active compound, such as a compound of formula I.

The term "prodrug" as used herein represents compounds which are transformed *in vivo* to the parent compound, for example, by hydrolysis in blood. An example of such a prodrug is a pharmaceutically acceptable ester of a carboxylic acid. A thorough discussion is provided in T. Higuchi and V. Stella, Pro-drugs as Novel Delivery Systems, Vol. 14 of the A.C.S. Symposium Series, Edward B. Roche, ed., Bioreversible Carriers in Drug Design, American Pharmaceutical Association and Pergamon Press, 1987; H Bundgaard, ed, Design of Prodrugs, Elsevier, 1985; and Judkins, et al. Synthetic Communications, 26(23), 4351-4367 (1996); and The organic chemistry of drug design and drug action by Richard B Silverman in particular pages 497 to 546; each of which is incorporated herein by reference. Another example of class of prodrugs comprise an acyl hydrazone on a keto group. See, for example, the 6-maleimidocaproyl)hydrazone derivative of doxorubicin, described in R. Graeser et al., "INNO-206, the (6-maleimidocaproyl hydrazone derivative of doxorubicin), shows superior antitumor efficacy compared to doxorubicin in different tumor xenograft models and in an orthotopic pancreas carcinoma model", 2009, Investigational New Drugs, 28(1):14-9, DOI 10.1007/s10637-008-9208-2, incorporated herein by reference. Compounds of the disclosure (such as compounds of formula I) comprise a keto group (such as at C13) that may be converted to a hydrazone to provide prodrugs thereof. Compounds of the disclosure (such as compounds of formula I) comprise hydroxy groups that may be esterified to provide prodrugs thereof.

The term "pharmaceutically acceptable salts" is meant to include salts of the active compounds which are prepared with relatively nontoxic acids or bases, depending on the particular substituents found on the compounds described herein. When compounds contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, ammonium, organic amino, or magnesium salt, or a similar salt. When compounds contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, maleic, malonic, benzoic, succinic, suberic, fumaric, lactic, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like (see, for example, Berge et al., "Pharmaceutical Salts", Journal of Pharmaceutical Science, 1977, 66, 1-19). Certain specific compounds of the present invention contain both basic and acidic functionalities that allow the compounds to be converted into either base or acid addition salts.

Thus, the compounds of the present disclosure may exist as salts with pharmaceutically acceptable acids. The present invention includes such salts. Examples of such salts include hydrochlorides, hydrobromides, sulfates, methanesulfonates, nitrates, maleates, acetates, citrates, fumarates, tartrates (e.g. (+)-tartrates, (-)-tartrates or mixtures thereof including racemic mixtures, succinates, benzoates and salts with amino acids such as glutamic acid. These salts may be prepared by methods known to those skilled in the art.

The neutral forms of the compounds are preferably regenerated by contacting the salt with a base or acid and isolating the parent compound in the conventional manner. The parent form of the compound differs from the various salt forms in certain physical properties, such as solubility in polar solvents.

Certain compounds of the present invention can exist in unsolvated forms as well as solvated forms, including hydrated forms. In general, the solvated forms are equivalent to unsolvated forms and are encompassed within the scope of the present invention. Certain compounds of the present invention may exist in multiple crystalline or amorphous forms. In general, all physical forms are equivalent for the uses contemplated by the present invention and are intended to be within the scope of the present invention.

Certain compounds of the present invention possess asymmetric carbon atoms (optical centres) or double bonds; the racemates, diastereomers, tautomers, geometric isomers and individual isomers are encompassed within the scope of the present invention. For example, compounds of formula I comprise asymmetric carbon atoms in the saccharide moiety, such as at the C that is attached to OR³. The compounds of the present invention do not include those which are known in the art to be too unstable to synthesize and/or isolate.

The compounds of the present invention may also contain unnatural proportions of atomic isotopes at one or more of the atoms that constitute such compounds. For example, the compounds may be radiolabelled with radioactive isotopes, such as for example tritium (³H), iodine-125 (¹²⁵I) or carbon-14 (¹⁴C). All isotopic variations of the compounds of the present invention, whether radioactive or not, are encompassed within the disclosure and invention.

### COMPOUNDS

In one aspect, the invention provides compounds of formula I as previously described or a pharmaceutically acceptable salt, stereoisomer, solvate, or prodrug thereof.

In an embodiment, R¹ is an unsubstituted C₁₋₄ alkyl. R¹ may be selected from CH₃, CH₂CH₃, CH(CH₃)₂ and CH₂CH₂CH₃. R¹ may be selected from CH₃ and CH₂CH₃. R¹ may be CH₃. R¹ may be CH₂CH₃.

In an embodiment, R² is an unsubstituted C₁₋₄ alkyl. R² may be selected from CH₃, CH₂CH₃, CH(CH₃)₂ and CH₂CH₂CH₃. R² may be selected from CH₃ and CH₂CH₃. R² may be CH₃. R² may be CH₂CH₃.

In an embodiment, R¹ is CH₃ and R² is CH₃.

In an embodiment, R¹ and / or R² is a substituted C₁₋₄ alkyl. The or each substituted C₁₋₄ alkyl may be of formula II: 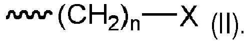 n is 1, 2, 3 or 4, e.g. n is 2 or 3. X is OH, halogen, or -OSO₂R⁵. R⁵ is a C₁₋₄ alkyl or aryl optionally substituted by C₁₋₄ alkyl, nitro, amino, methoxy, or halogen. For example, n may be 2 or 3, and X may be OH or halogen. Where both R¹ and R² are of formula II, each moiety of formula II may be the same, or each moiety of formula II may be different.

In an embodiment, R³ is selected from H, and 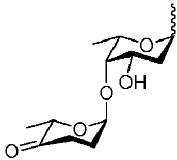 where 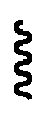 designates the point of attachment to the rest of the compound. R³ may be H. R³ may be

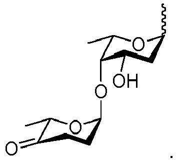

In an embodiment, R⁴ is H. In another embodiment, R⁴ is OH.

In an embodiment, the compound of the invention is selected from: 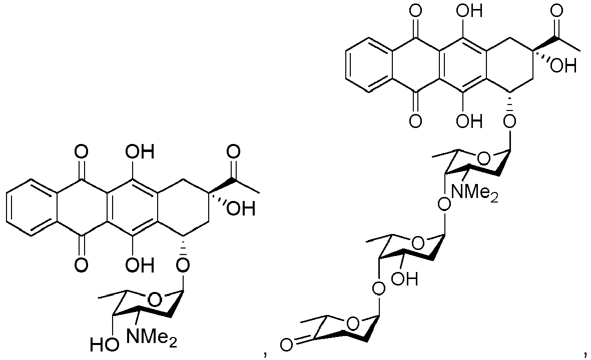 or a pharmaceutically acceptable salt, stereoisomer, solvate, or prodrug thereof.

In an embodiment the compound has an IC₅₀ of less than about 300 nM against cell line K562 and wherein the fraction of broken DNA damage in the K562 cells is less than about 0.10. In an embodiment the compound has an IC₅₀ of less than about 200 nM against cell line K562 and wherein the fraction of broken DNA damage in the K562 cells is less than about 0.10. For example, the IC₅₀ may be less than about 150 nM against cell line K562, e.g. the IC₅₀ may be less than about 125 nM. Preferably, the IC₅₀ may be less than about 100 nM against cell line K562, e.g. the IC₅₀ may be not more than about 80 nM. For example, the fraction of broken DNA damage in the K562 cells may be less than about 0.05.

The IC₅₀ of the compound with a given cell line may be assessed in accordance with "Assay 1: Short-term growth inhibition viability assay" provided in the assays of the present disclosure. The cell line to be assessed represent the cells to be used in the given assay. The fraction of broken DNA damage in given cells or evaluation of the double strand DNA response by yH2AX levels in given cells for the compound may be assessed using "Assay 2: Constant-field gel electrophoresis and Assay 3: Western blot". The cell line or cells to be assessed represent the cells to be used in the given assay.

### FORMULATIONS AND ADMINISTRATION

Compounds of the invention may be administered orally, topically, intravenously, subcutaneously, buccally, rectally, dermally, nasally, tracheally, bronchially, by any other parenteral route, as an oral or nasal spray or via inhalation. The compounds may be administered in the form of pharmaceutical preparations comprising prodrug or active compound either as a free compound or, for example, a pharmaceutically acceptable nontoxic organic or inorganic acid or base addition salt, in a pharmaceutically acceptable dosage form. Depending upon the disorder and patient to be treated and the route of administration, the compositions may be administered at varying doses.

Typically, therefore, the pharmaceutical compounds of the invention may be administered orally, topically, or parenterally ("parenterally" as used herein, refers to modes of administration which include intravenous, intramuscular, intraperitoneal, intrasternal, subcutaneous and intraarticular injection and infusion) to a host to obtain a proteaseinhibitory effect. In the case of larger animals, such as humans, the compounds may be administered alone or as compositions in combination with pharmaceutically acceptable diluents, excipients or carriers.

Actual dosage levels of active ingredients in the pharmaceutical formulations and pharmaceutical compositions of this invention may be varied so as to obtain an amount of the active compound(s) that is effective to achieve the desired therapeutic response for a particular patient, compositions and mode of administration. The selected dosage level will depend upon the activity of the particular compound, the route of administration, the severity of the condition being treated and the condition and prior medical history of the patient being treated. However, it is within the skill of the art to start doses of the compound at levels lower than required to achieve the desired therapeutic effect and to gradually increase the dosage until the desired effect is achieved.

In the treatment, prevention, control, amelioration, or the like of cancer, an appropriate dosage level may generally be about 0.01 to 500 mg per kg patient body weight per day which can be administered in single or multiple doses. The dosage level may be be about 0.1 to about 100 mg/kg per day; more preferably about 0.5 to about 50 mg/kg per day. For oral administration, the compositions may be provided in the form of tablets containing 1.0 to 1000 milligrams of the active ingredient, particularly 1.0, 5.0, 10.0, 15.0, 20.0, 25.0, 50.0, 75.0, 100.0, 150.0, 200.0, 250.0, 300.0, 400.0, 500.0, 600.0, 750.0, 800.0, 900.0 and 1000.0 milligrams of the active ingredient for the symptomatic adjustment of the dosage to the patient to be treated. The compounds may be administered on a regimen of 1 to 4 times per day, e.g. once or twice per day. The compounds may be administered on a regimen comprising a daily dose for several consecutive days. The dosage regimen may be adjusted to provide the optimal therapeutic response.

According to a further aspect of the invention there is thus provided a pharmaceutical formulation or composition including a compound of the invention (such as a compound of the first aspect, or an embodiment thereof). The compound may be in admixture with a pharmaceutically acceptable adjuvant, diluent or carrier. In an embodiment, the formulation or composition is an oral formulation. For example, the formulation or composition may be formulated for oral administration.

Solid dosage forms for oral administration include capsules, tablets, pills, powders and granules. In such solid dosage forms, the active compound is typically mixed with at least one inert, pharmaceutically acceptable excipient or carrier, such as sodium citrate or dicalcium phosphate and/or one or more: a) fillers or extenders, such as starches, lactose, sucrose, glucose, mannitol and silicic acid; b) binders, such as carboxymethylcellulose, alginates, gelatine, polyvinylpyrrolidone, sucrose and acacia; c) humectants, such as glycerol; d) disintegrating agents, such as agar-agar, calcium carbonate, potato or tapioca starch, alginic acid, certain silicates and sodium carbonate; e) solution retarding agents, such as paraffin; f) absorption accelerators, such as quaternary ammonium compounds; g) wetting agents, such as acetyl alcohol and glycerol monostearate; h) absorbents, such as kaolin and bentonite clay and i) lubricants, such as talc, calcium stearate, magnesium stearate, solid polyethylene glycols, sodium lauryl sulfate and mixtures thereof. In the case of capsules, tablets and pills, the dosage form may also comprise buffering agents. Solid compositions of a similar type may also be employed as fillers in soft and hard-filled gelatin capsules using such excipients as lactose or milk sugar as well as high molecular weight polyethylene glycol, for example.

Oral formulations may contain a dissolution aid. Examples of dissolution aids include non-ionic surface active agents, such as sucrose fatty acid esters, glycerol fatty acid esters, sorbitan fatty acid esters (e.g. sorbitan trioleate), polyethylene glycol, polyoxyethylene hydrogenated castor oil, polyoxyethylene sorbitan fatty acid esters, polyoxyethylene alkyl ethers, methoxypolyoxyethylene alkyl ethers, polyoxyethylene alkylphenyl ethers, polyethylene glycol fatty acid esters, polyoxyethylene alkylamines, polyoxyethylene alkyl thioethers, polyoxyethylene polyoxypropylene copolymers, polyoxyethylene glycerol fatty acid esters, pentaerythritol fatty acid esters, propylene glycol monofatty acid esters, polyoxyethylene propylene glycol monofatty acid esters, polyoxyethylene sorbitol fatty acid esters, fatty acid alkylolamides, and alkylamine oxides; bile acid and salts thereof (e.g. chenodeoxycholic acid, cholic acid, deoxycholic acid, dehydrocholic acid and salts thereof, and glycine or taurine conjugate thereof); ionic surface active agents, such as sodium laurylsulfate, fatty acid soaps, alkylsufonates, alkylphosphates, ether phosphates, fatty acid salts of basic amino acids; triethanolamine soap, and alkyl quaternary ammonium salts; and amphoteric surface active agents, such as betaines and aminocarboxylic acid salts.

The solid dosage forms of tablets, dragees, capsules, pills, and granules can be prepared with coatings and shells such as enteric coatings and other coatings well known in the pharmaceutical formulating art. They may optionally contain opacifying agents and may also be of a composition such that they release the active ingredient(s) only, or preferentially, in a certain part of the intestinal tract, and/or in delayed fashion. Examples of embedding compositions include polymeric substances and waxes.

Liquid dosage forms for oral administration include pharmaceutically acceptable emulsions, solutions, suspensions, syrups and elixirs. In addition to the active compounds, the liquid dosage forms may contain inert diluents commonly used in the art such as water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor and sesame oils), glycerol, tetrahydrofurfuryl alcohol, polyethylene glycols and fatty acid esters of sorbitan and mixtures thereof. Besides inert diluents, the oral compositions may also include adjuvants such as wetting agents, emulsifying and suspending agents, sweetening, flavouring and perfuming agents. Suspensions, in addition to the active compounds, may contain suspending agents such as ethoxylated isostearyl alcohols, polyoxyethylene sorbitol and sorbitan esters, microcrystalline cellulose, aluminium metahydroxide, bentonite, agar-agar, and traganacanth and mixtures thereof.

Pharmaceutical formulations or compositions of this invention for parenteral injection may comprise pharmaceutically acceptable sterile aqueous or non-aqueous solutions, dispersions, suspensions or emulsions as well as sterile powders for reconstitution into sterile injectable solutions or dispersions just prior to use. Examples of suitable aqueous and non-aqueous carriers, diluents, solvents or vehicles include water, ethanol, polyols (such as glycerol, propylene glycol, polyethylene glycol and the like), and suitable mixtures thereof, vegetable oils (such as olive oil) and injectable organic esters, such as ethyl oleate. Proper fluidity can be maintained, for example, by the use of coating materials, such as lecithin, by the maintenance of the required particle size in the case of dispersions and by the use of surfactants.

These compositions may also contain adjuvants such as preservative, wetting agents, emulsifying agents and dispersing agents. Inhibition of the action of microorganisms may be ensured by the inclusion of various antibacterial and antifungal agents, for example, paraben, chlorobutanol or phenol sorbic acid. It may also be desirable to include isotonic agents, such as sugars or sodium chloride, for example. Prolonged absorption of the injectable pharmaceutical form may be brought about by the inclusion of agents (for example, aluminium monostearate and gelatine) which delay absorption.

Compositions for rectal or vaginal administration may be in the form of suppositories which can be prepared by mixing the compounds of this invention with suitable non-irritating excipients or carriers such as cocoa butter, polyethylene glycol or a suppository wax which are solid at room temperature but liquid at body temperature and therefore melt in the rectum or vaginal cavity and release the active compound.

Insofar as they do not interfere with the activity of the compounds, the compositions and formulations according to the present subject matter may contain other active agents intended, in particular, for use in treating a cancer. Such other active agents may include one or more additional chemotherapeutic agents. Such other active agents may include one or more agents to ameliorate cancer symptoms, or to ameliorate the undesired side effects of chemotherapy.

The formulations according to the present subject matter may also contain inactive components. Suitable inactive components are well known in the art and are described in standard textbooks, such as Goodman and Gillman's: The Pharmacological Bases of Therapeutics, 8thEd., Gilman et al, Eds. Pergamon Press (1990), and Remington's Pharmaceutical Sciences, 17th Ed., Mack Publishing Co., Easton, Pa. (1990), both of which are incorporated by reference herein in their entirety.

The formulations and compositions may be used in combination with an additional pharmaceutical dosage form to enhance their effectiveness in treating any of the disorders described herein. In this regard, the present formulations and compositions may be administered as part of a regimen additionally including any other pharmaceutical and/or pharmaceutical dosage form known in the art as effective for the treatment of any of these disorders.

### USES

Anthracyclines can be highly effective anti-cancer drugs, however their clinical application is hampered by treatment-limiting side effects (such as cardiotoxicity, secondary tumor formation and infertility) and drug resistance. Cardiotoxicity is one of the main adverse effect which emerges in a cumulative manner and is restricting treatment regimens as a consequence. The compounds of the invention comprise anthracycline glycosides that are useful in the treatment of cancer. The compounds of the invention may minimize (or at least reduce) the severe side effects, such as cytotoxicity. The compounds of the invention may provide good activity in cells lines that are resistant to some other anthracyclines, such as doxorubicin. In particular, the compounds of the invention may provide good activity in cell lines that demonstrate resistance to some other anthracyclines, such as doxorubicin, via ATP binding cassette subfamily B member 1 (ABCB1) overexpression.

Anthracycline compounds are believed to provide activity by more than one mechanism. Most, anthracycline drugs cause DNA double-strand breaks by inhibition or poisoning of topoisomerase II. This mode of action was thought to be the main reason for the remarkable effectiveness of these drugs. It was recently determined that DNA damage is not the only mode of action for most anthracycline variants. All clinically used anthracyclines induce chromatin damage upon DNA intercalation and subsequent eviction of histones. Furthermore, it was shown that the combination of DNA damage and chromatin damage, as exerted by doxorubicin, epirubicin, daunorubicin and idarubicin, results in the major side effects reported for these compounds. In contrast, aclarubicin solely induces chromatin damage and is not cardiotoxic nor induces therapy related malignancies.

As indicated in the examples, exemplary compounds of formula I induce significant chromatin damage, but do not cause significant DNA double-strand breaks. Thus similarly to aclarubicin, compounds of the invention may avoid or minimise side effects (such as cardiotoxicity and secondary tumor formation). Accordingly, exemplary compounds of formula I may be useful in patients with higher cardiotoxicity risk, who are excluded from treatment with conventional anthracycline anti-cancer treatments. In addition, compounds of the invention have been demonstrated to have better anticancer activity than doxorubicin, e.g. in human myelogenous leukemic cell line K562, human melanoma cell line MelJuSo and human osteosarcoma cell line U2OS.

Compounds of the invention may be used to treat conditions such as cancer. The cancer may be selected from Acute Lymphoblastic Leukemia (ALL), Acute Myeloid Leukemia (AML), Adrenocortical Carcinoma, Anal Cancer, Appendix Cancer, Atypical Teratoid/Rhabdoid Tumor, Basal Cell Carcinoma, Bile Duct Cancer, Biliary Cancer, Bladder Cancer, Bone Cancer, Brain Tumor, Astrocytoma, Brain and Spinal Cord Tumor, Brain Stem Glioma, Central Nervous System Atypical Teratoid/Rhabdoid Tumor, Central Nervous System Embryonal Tumor, Breast Cancer, Bronchial Tumor, Burkitt Lymphoma, Carcinoid Tumor, Cervical Cancer, Childhood Cancer, Chordoma, Chronic Lymphocytic Leukemia (CLL), Chronic Myelogenous Leukemia (CML), Chronic Myeloproliferative Disorder, Colon Cancer, Colorectal Cancer, Craniopharyngioma, Cutaneous T-Cell Lymphoma, Ductal Carcinoma In Situ (DCIS), Embryonal Tumors, Endometrial Cancer, Ependymoblastoma, Ependymoma, Esophageal Cancer, Esthesioneuroblastoma, Ewing Sarcoma, Extracranial Germ Cell Tumor, Extragonadal Germ Cell Tumor, Extrahepatic Bile Duct Cancer, Eye Cancer, Fibrous Histiocytoma of Bone, Gallbladder Cancer, Gastric Cancer, Gastrointestinal Carcinoid Tumor, Gastrointestinal Stromal Tumors (GIST), Extracranial Germ Cell Tumor, Extragonadal Germ Cell Tumor, Ovarian Germ Cell Tumor, Gestational Trophoblastic Tumor, Glioma, Hairy Cell Leukemia, Head and Neck Cancer, Heart Cancer, Hepatocellular Cancer, Hodgkin Lymphoma, Hypopharyngeal Cancer, Intraocular Melanoma, Islet Cell Tumors, Kaposi Sarcoma, Kidney Cancer, Langerhans Cell Histiocytosis, Laryngeal Cancer, Liver Cancer, Lobular Carcinoma In Situ (LCIS), Lung Cancer, Lymphoma, AIDS-Related Lymphoma, Male Breast Cancer, Medulloblastoma, Medulloepithelioma, Melanoma, Merkel Cell Carcinoma, Malignant Mesothelioma, Metastatic Squamous Neck Cancer with Occult Primary, Midline Tract Carcinoma Involving *NUT* Gene, Mouth Cancer, Multiple Endocrine Neoplasia Syndrome, Multiple Myeloma/Plasma Cell Neoplasm, Mycosis Fungoides, Myelodysplastic Syndrome, Myelodysplastic/Myeloproliferative Neoplasm, Multiple Myeloma, Nasal Cavity Cancer, Paranasal Sinus Cancer, Nasopharyngeal Cancer, Neuroblastoma, Non-Hodgkin Lymphoma, Non-Small Cell Lung Cancer, Oral Cancer, Oral Cavity Cancer, Lip Cancer, Oropharyngeal Cancer, Osteosarcoma, Ovarian Cancer, Pancreatic Cancer, Papillomatosis, Paraganglioma, Parathyroid Cancer, Penile Cancer, Pharyngeal Cancer, Pheochromocytoma, Pineal Parenchymal Tumors of Intermediate Differentiation, Pineoblastoma, Pituitary Tumor, Plasma Cell Neoplasm, Pleuropulmonary Blastoma, Breast Cancer, Primary Central Nervous System (CNS) Lymphoma, Prostate Cancer, Rectal Cancer, Renal Cell Cancer, Renal Pelvis Cancer, Ureter Cancer, Retinoblastoma, Rhabdomyosarcoma, Salivary Gland Cancer, Sézary Syndrome, Skin Cancer, Small Cell Lung Cancer, Small Intestine Cancer, Soft Tissue Sarcoma, Squamous Cell Carcinom, Supratentorial Primitive Neuroectodermal Tumors, T-Cell Lymphoma, Testicular Cancer, Throat Cancer, Thymoma, Thymic Carcinoma, Thyroid Cancer, Transitional Cell Cancer of the Renal Pelvis and Ureter, Gestational Trophoblastic Tumor, Urethral Cancer, Uterine Cancer, Uterine Sarcoma, Waldenström Macroglobulinemia, or Wilms Tumor.

The cancer may be a leukemia, a breast cancer, or a melanoma. The cancer may be a leukemia selected from Acute Lymphoblastic Leukemia (ALL), Acute Myeloid Leukemia (AML), Chronic Lymphocytic Leukemia (CLL), Chronic Myelogenous Leukemia (CML), and Hairy Cell Leukemia.

An embodiment provides a compound of the invention for use in the treatment of cancer, or a formulation or composition of the invention, for use in the treatment of cancer. The cancer may be a cancer disclosed herein.

The cancer may be selected from a haematological cancer or a solid tumor. The haematological cancer may be a leukemia, such as acute myeloid leukemia (AML) or acute lymphatic leukemia (ALL). The solid tumor may be a breast cancer or a melanoma, e.g. the solid tumor may be a breast cancer.

Another embodiment provides a method for the treatment of a cancer in a mammal in need of said treatment. The method comprises administering to the mammal a therapeutically effective amount of any of the compounds or any of the pharmaceutical compositions or formulations described herein. The mammal may be a human and / or the mammal may have higher cardiotoxicity risk.

The cancer may be a cancer disclosed herein. The cancer may be selected from a haematological cancer or a solid tumor. The haematological cancer may be a leukemia, such as acute myeloid leukemia (AML) or acute lymphatic leukemia (ALL). The solid tumor may be a breast cancer or a melanoma, e.g. the solid tumor may be a breast cancer.

Another embodiment provides use of a compound of the invention in the manufacture of a medicament for use in the treatment of cancer.

The cancer may be a cancer disclosed herein. The cancer may be selected from a haematological cancer or a solid tumor. The haematological cancer may be a leukemia, such as acute myeloid leukemia (AML) or acute lymphatic leukemia (ALL). The solid tumor may be a breast cancer or a melanoma, e.g. the solid tumor may be a breast cancer.

### Assays

The activity of the compounds on specific cells may be assessed using a variety of assays. Exemplary assays are described below.

### Assay 1: Short-term growth inhibition viability assay

Cells were seeded into 96-well format (e.g. 2000 cells/well for K562, Mjs, or U2O cells). 24 hours after seeding, cells were treated with indicated compounds for 2 hours at various concentrations. Subsequently, compounds were removed, and cells were left to grow for an additional 72 hours. Cell viability was measured using the CellTiter-Blue viability assay (Promega). Relative survival was normalized to the untreated control and corrected for background signal.

### Assay 2: Constant-field gel electrophoresis (CFGE)

Cells were seeded into 12-well format (e.g. 250.000 cells/well for for K562, or Mjs), treated with indicated drugs at 10 µM for 2 hours. Subsequently, drugs were removed by extensive washing and cells were collected and processed immediately for the assays. DNA double strand breaks were quantified by constant-field gel electrophoresis as described in Wlodek D., Banáth J., & Olive P. L., International Journal of Radiation Biology, 1991, 60:5, 779-790*.* Images were quantified using imaged software.

### Assay 3: Western blot

Cells were seeded into 12-well format (e.g. 250.000 cells/well for for K562, or Mjs), treated with indicated drugs at 10 µM for 2 hours. Subsequently, drugs were removed by extensive washing and cells were collected and processed immediately for the assays. Cells were lysed directly in SDS-sample buffer (2%SDS, 10% glycerol, 5% β-mercaptoethanol, 60mM Tris-HCl pH 6.8 and 0.01% bromophenol blue). Samples were separated by SDS-PAGE and transferred to a PVDF membrane (Immobilon-P, 0.45µm, Millipore). Blocking of the filters and antibody incubations were done in PBS supplemented with 0.1 (v/v)% Tween and 5% (w/v) milk powder (Skim milk powder, LP0031, Oxiod). Blots were imaged by the Odyssey Classic imager (Li-Cor). Intensity of bands was quantified using imaged or Image Studio software. For CFGE: DNA double strand breaks were quantified by constant-field gel electrophoresis as described in Wlodek D., Banáth J., & Olive P. L., International Journal of Radiation Biology, 1991, 60:5, 779-790*.* Images were quantified using imaged software.

Primary antibodies used for Western blotting: γH2AX (1:1000, 05-036, Millipore), β-actin (1:10000, A5441, Sigma). Secondary antibody used for blotting: IRDye 800CW goat anti-mouse IgG (H+L) (926-32210, Li-COR, 1:10000).

### Assay 4: Microscopy analysis

For PAGFP-H2A photoactivation and time-lapse confocal imaging, cells were seeded in a 35mm glass bottom petri dish (Poly-dlysine-Coated, MatTek Corporation), and imaged 16 hours later as described in Pang, B., Qiao, X., Janssen, L. et al. Nat Commun 4, 2013, 1908 for one hour following addition of 10µM of the indicated drugs. Time-lapse confocal imaging was performed on a Leica SP8 confocal microscope system 63x lens, equipped with a climate chamber. Movies were quantified using imaged software.

### Examples

### Example 1: Compound Synthesis

Doxorubicin (**1**) was obtained from Accord Healthcare Limited, UK, aclarubicin (**2**) (sc-200160) was purchased from Santa Cruz Biotechnology (USA), daunorubicin (**15**) was obtained from Sanofi, idarubicin (**17**) was obtained from Pfizer, etoposide [E] was obtained from TEVA Pharmachemie (the Netherlands).

### Synthesis of Anthracycline analogue compounds 18, 25 and 26

All reagents were of commercial grade and used as received. Traces of water from reagents were removed by co-evaporation with toluene in reactions that required anhydrous conditions. All moisture/oxygen sensitive reactions were performed under an argon atmosphere. DCM used in the glycosylation reactions was dried with flamed 4Å molecular sieves before being used. Reactions were monitored by TLC analysis with detection by UV (254 nm) and where applicable by spraying with 20% sulfuric acid in EtOH or with a solution of (NH₄)₆Mo₇O₂₄·4H₂O (25 g/L) and (NH₄)₄Ce(SO₄)₄·2H₂O (10 g/L) in 10% sulfuric acid (aq.) followed by charring at ~150 °C. Flash column chromatography was performed on silica gel (40-63µm). ¹H and ¹³C spectra were recorded on a Bruker AV 400 and Bruker AV 500 in CDCl₃, CD₃OD, pyridine-d5 or D₂O. Chemical shifts (δ) are given in ppm relative to tetramethylsilane (TMS) as internal standard (¹H NMR in CDCl₃) or the residual signal of the deuterated solvent. Coupling constants (J) are given in Hz. All ¹³C spectra are proton decoupled. Column chromatography was carried out using silica gel (0.040-0.063 mm). Size-exclusion chromatography was carried out using Sephadex LH-20, using DCM:MeOH (1:1, v/v) as the eluent. Neutral silica was prepared by stirring regular silica gel (0.040-0.063 mm) in aqueous ammonia, followed by filtration, washing with water and heating at 150°C overnight. High-resolution mass spectrometry (HRMS) analysis was performed with a LTQ Orbitrap mass spectrometer (Thermo Finnigan), equipped with an electronspray ion source in positive mode (source voltage 3.5 kV, sheath gas flow 10 mL/min, capillary temperature 250 °C) with resolution R = 60000 at m/z 400 (mass range m/z = 150 - 2000) and dioctyl phthalate (m/z = 391.28428) as a "lock mass", or with a Synapt G2-Si (Waters) , equipped with an electronspray ion source in positive mode (ESI-TOF), injection via NanoEquity system (Waters), with LeuEnk (m/z = 556.2771) as "lock mass". Eluents used: MeCN:H₂O (1:1 v/v) supplemented with 0.1% formic acid. The high-resolution mass spectrometers were calibrated prior to measurements with a calibration mixture (Thermo Finnigan).

### General procedure A: N-cyclic doxorubicins

To a solution of doxorubicin·HCl in DMF (0.033M) were added triethylamine (3 eq) and the corresponding diiodoalkane or diiodoether (18 eq). The mixture was allowed to stir for 5 days, or until LCMS showed the disappearance of the starting material. It was then poured into H₂O, extracted with CHCl₃ repetitively, dried over Na₂SO₄ and concentrated in vacuo. Column chromatography on neutral silica (MeOH:DCM) gave the title cyclic amines.

### General procedure C: Glycosylation of alkynylbenzoate donors

To a solution of the alkynylbenzoate donor and anthracyclinone acceptor (1.5 eq) in DCM (0.05 M), were added activated molecular sieves (4Å) and the mixture was stirred for 30 minutes. Subsequently, a freshly prepared 0.1 M DCM solution of PPh₃AuNTf₂ (prepared by stirring 1:1 PPh₃AuCl and AgNTf₂ in DCM for 30 minutes) (0.1 eq) in DCM was added dropwise. After stirring 30 minutes, the mixture was filtered and concentrated *in vacuo.* Column chromatography gave the title anthracyclinone saccharides.

### General procedure D: N-Alloc removal

A solution of the allyloxycarbamate and N,N-dimethylbarbituric acid (4.5 eq) in DCM (0.01 M) was degassed for 5 minutes. Then, Pd(PPh3)4 (0.05 eq) was added and the mixture was allowed to stir for 30 minutes. The reaction was then directly subjected to column chromatography to give the title amines.

### Synthesis of N,N-dimethylidarubicin (18)

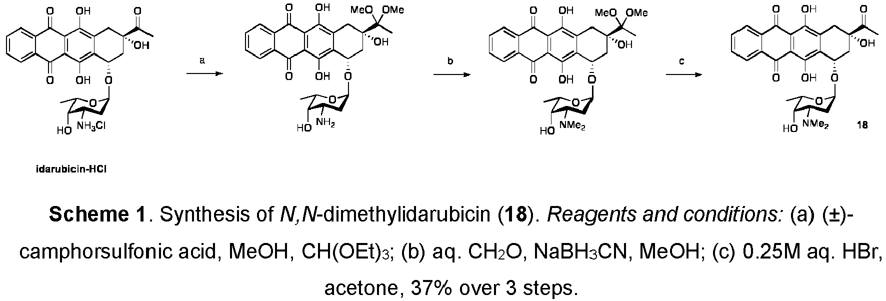

To a solution of idarubicin hydrochloride (50.0 mg, 0.0936 mmol) in MeOH (3 mL) and trimethylorthoformate (1 mL) was added (±)-camphorsulfonic acid (50 mg, 0.215 mmol, 2.3 eq) and the mixture was stirred overnight. It was then poured into sat. aq. NaHCO₃ (10 mL) and extracted with CHCl₃ (20 mL, 2x). The organic layers were dried over Na₂SO₄ and concentrated *in vacuo* to give the crude dimethyl acetal as a yellow solid.

This was redissolved in a mixture of MeOH (3 mL), DCM (2 mL) and 37% aq. CH₂O (0.7 mL, stabilised with 10-15% MeOH), after which NaBH₃CN (29.3 mg, 0.47 mmol, 5 eq) was added. After stirring for 2 hours, the reaction was poured into sat. aq. NaHCOs (10 mL). This was then extracted with CHCl₃ (20 mL), dried over Na₂SO₄ and concentrated *in vacuo.* This procedure was performed twice to afford the crude dimethylamine as a yellow solid.

A solution of the above acetal in acetone (8 mL) and 0.25M aq. HBr (8 mL) was stirred for 1 hour, after which it was diluted with H₂O (20 mL). This was then washed with DCM (30 mL, 3x) basified by addition of sat. aq. NaHCO₃ (10 mL) and extracted with CHCl₃ (30 mL, 2x). Drying over Na₂SO₄ and concentration *in vacuo* afforded the title compound as a yellow solid (18 mg, 34 umol, 37% over 3 steps). ¹H NMR (400 MHz, CDCl₃) δ 14.05 - 12.81 (m, 2H), 8.32 (ddd, *J* = 6.3, 5.0, 3.3 Hz, 2H), 7.87 - 7.80 (m, 2H), 5.66 - 5.47 (m, 1H), 5.27 (dd, *J* = 4.1, 2.1 Hz, 1H), 4.77 (s, 1H), 4.09 - 4.02 (m, 1H), 3.76 - 3.70 (m, 1H), 3.29 - 2.94 (m, 2H), 2.44 (s, 3H), 2.38 (dt, *J* = 14.8, 2.2 Hz, 1H), 2.24 (s, 6H), 2.10 (dd, *J* = 14.8, 4.2 Hz, 1H), 1.92 - 1.76 (m, 2H), 1.40 (d, *J* = 6.6 Hz, 3H). ¹³C NMR (101 MHz, CDCl₃) δ 211.8, 186.9, 186.7, 156.8, 156.5, 136.1, 134.7, 134.0, 133.6, 133.4, 127.2, 127.1, 111.5, 110.8, 101.1, 77.5, 77.2, 77.0, 76.8, 69.8, 66.8, 66.1, 59.8, 42.1, 35.0, 33.7, 29.8, 28.7, 24.9, 17.2. HRMS: [M + H]⁺ calculated for C₂₈H₃₁NO₉526.2077; found 526.2069.

### Synthesis of idarubicinone trisaccharides (25) and (26)

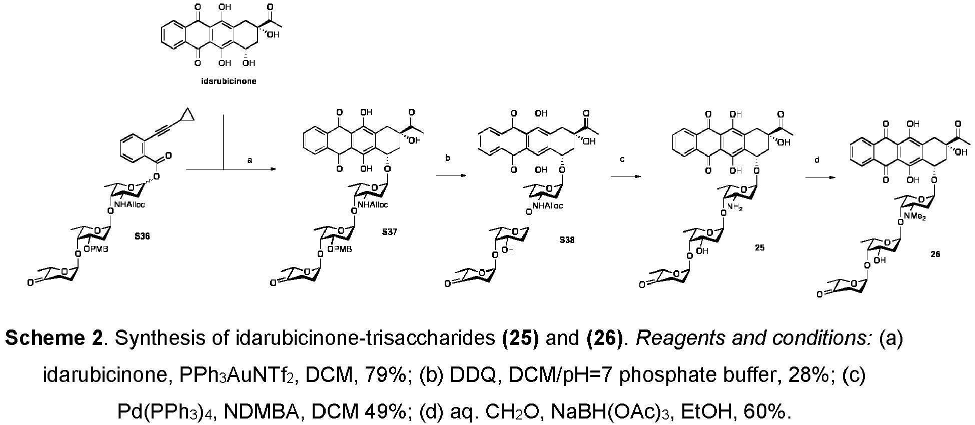

### 7-[2,3-Dideoxy-4-ulo-α-L-fucopyranosyl-2-deoxy-3-O-p-methoxybenzyl-α-L-fucopyranosyl-(1→4)-3-N-allyloxycarbonyl-2,3-dideoxy-α-L-fucopyranoside]-idarubicinone (S37)

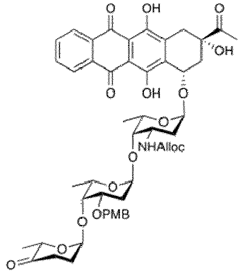

According to general procedure C, glycosyl donor S36 (synthesised as per Wander D. P. A., van der Zanden S. Y. et al. J Med Chem 2020, 63 (21), 12814-12829) (278 mg, 0.362 mmol) was glycosylated to idarubicinone (160 mg, 0.434 mmol, 1.2 eq) in DCM (14.4 mL). Column chromatography (1:99 - 8:92 acetone:DCM) afforded the title compound as an orange solid (270 mg, 0.286 mmol, 79%). ¹H NMR (400 MHz, CDCl₃) δ 13.59 (d, *J* = 1.2 Hz, 1H), 13.34 (s, 1H), 8.35 (ddt, *J* = 6.9, 5.8, 3.4 Hz, 2H), 7.85 (ddd, *J* = 6.3, 3.3, 1.0 Hz, 2H), 7.26 (d, *J* = 8.7 Hz, 2H), 6.96 - 6.82 (m, 2H), 6.12 (d, *J* = 8.0 Hz, 1H), 5.84 (ddt, *J* = 16.3, 10.8, 5.6 Hz, 1H), 5.51 (d, *J* = 3.7 Hz, 1H), 5.30 (s, 1H), 5.27 - 5.12 (m, 2H), 5.10 (t, *J* = 4.4 Hz, 1H), 4.99 (s, 1H), 4.73 - 4.41 (m, 6H), 4.23 - 4.14 (m, 1H), 4.03 (t, *J* = 6.6 Hz, 1H), 3.99 (d, *J* = 2.8 Hz, 1H), 3.97 - 3.85 (m, 2H), 3.82 (s, 3H), 3.57 (s, 1H), 3.27 (d, *J* = 18.6 Hz, 1H), 2.99 (d, *J* = 19.0 Hz, 1H), 2.60 (ddd, *J* = 15.0, 8.7, 5.5 Hz, 1H), 2.43 (d, *J* = 6.6 Hz, 4H), 2.37 - 2.23 (m, 2H), 2.24 - 2.03 (m, 4H), 1.94 (dd, *J* = 12.9, 4.5 Hz, 1H), 1.75 (td, *J* = 12.9, 3.9 Hz, 1H), 1.35 - 1.19 (m, 6H), 0.98 (d, *J* = 6.7 Hz, 3H). ¹³C NMR (101 MHz, CDCl₃) δ 212.2, 211.2, 187.0, 186.8, 159.3, 156.7, 156.6, 155.7, 136.1, 134.7, 133.6, 133.0, 130.4, 129.1, 127.3, 127.1, 117.6, 114.0, 111.6, 111.0, 101.5, 100.3, 98.0, 81.0, 75.1, 72.4, 71.9, 70.4, 69.2, 68.6, 68.1, 65.6, 55.4, 46.6, 35.1, 34.0, 33.9, 31.5, 31.1, 29.5, 25.0, 17.5, 17.3, 14.9. HRMS: [M + Na]⁺ calculated for C50H57NO17 966.3524; found 966.3514.

### 7-[2,3-Dideoxy-4-ulo-α-L-fucopyranosyl-2-deoxy-α-L-fucopyranosyl-(1→4)-3-N-allyloxycarbonyl-2,3-dideoxy-α-L-fucopyranoside]-idarubicinone (S38)

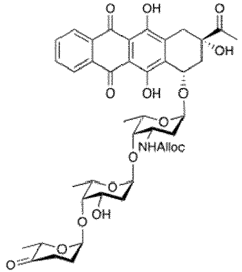

To a biphasic mixture compound S37 (270 mg, 0.286 mmol) in DCM (48 mL) and phosphate buffer (4.8 mL, pH=7) was added DDQ (325 mg, 1.43 mmol, 5 eq) at 0°C after which the mixture was stirred at that temperature for 5 hours. It was diluted with DCM, washed with H₂O four times, after which the organic layer was dried over Na₂SO₄ and concentrated *in vacuo.* Column chromatography (5:95 - 7:93 acetone:DCM) gave the title compound as a red solid (66 mg, 0.080 mmol, 28%). ¹H NMR (500 MHz, CDCl₃) δ 13.56 (s, 1H), 13.33 (s, 1H), 8.50 - 8.24 (m, 2H), 7.84 (dd, *J* = 5.9, 3.3 Hz, 2H), 6.05 (d, *J* = 7.9 Hz, 1H), 5.84 (ddt, *J* = 16.4, 10.8, 5.6 Hz, 1H), 5.51 (d, *J* = 3.7 Hz, 1H), 5.34 - 5.05 (m, 4H), 4.95 (d, *J* = 3.9 Hz, 1H), 4.59 (s, 1H), 4.55 - 4.40 (m, 3H), 4.20 (q, *J* = 6.4 Hz, 1H), 4.13 (dq, *J* = 13.7, 6.9 Hz, 2H), 3.95 - 3.84 (m, 1H), 3.75 (dd, *J* = 11.2, 6.2 Hz, 2H), 3.58 (s, 1H), 3.26 (dd, *J* = 19.1, 1.9 Hz, 1H), 2.98 (d, *J* = 19.0 Hz, 1H), 2.63 (s, 1H), 2.47 (dddd, *J* = 14.6, 12.0, 9.6, 5.4 Hz, 3H), 2.41 (s, 3H), 2.32 (d, *J* = 15.2 Hz, 1H), 2.22-2.01 (m, 4H), 2.01-1.85 (m, 2H), 1.78 (td, *J* = 12.9, 4.0 Hz, 1H), 1.41-1.21 (m, 9H). ¹³C NMR (126 MHz, CDCl₃) δ 212.2, 209.9, 187.0, 186.8, 156.8, 156.6, 155.7, 136.1, 134.6, 134.6, 133.8, 133.7, 133.6, 133.0, 127.2, 127.1, 117.6, 111.6, 110.9, 101.8, 101.6, 100.7, 100.3, 82.2, 81.2, 77.4, 77.2, 76.9, 76.8, 72.0, 69.5, 68.0, 67.9, 65.6, 65.1, 53.9, 46.7, 35.1, 34.4, 33.8, 33.5, 31.9, 31.5, 30.1, 29.8, 29.4, 27.6, 25.0, 17.4, 16.9, 14.9. HRMS: [M + Na]⁺ calculated for C42H49NO16 846.2949; found 846.2937.

### 7-[2,3-Dideoxy-4-ulo-α-L-fucopyranosyl-2-deoxy-α-L-fucopyranosyl-(1→4)-3-amino-2,3-dideoxy-α-L-fucopyranoside]-idarubicinone (25)

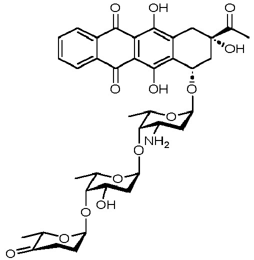

According to general procedure D, S38 (66 mg, 0.089 mmol) was deprotected. Size-exclusion chromatography (Sephadex LH-20, 1:1 DCM:MeOH v/v) gave the crude amine. Then, silica gel column chromatography (20:80 - 80:20 acetone:DCM) gave the free amine (29.2 mg, 39.4 umol, 49%). ¹H NMR (500 MHz, CDCl₃) δ 8.32 (s, 2H), 7.90 - 7.76 (m, 2H), 5.51 (s, 1H), 5.26 (s, 1H), 5.09 (t, *J* = 6.2 Hz, 1H), 5.01 (s, 1H), 4.48 (q, *J* = 6.7 Hz, 1H), 4.23 (d, *J* = 6.7 Hz, 1H), 4.13 (d, *J* = 6.8 Hz, 2H), 3.72 (s, 1H), 3.57 (s, 1H), 3.24 (d, *J* = 18.9 Hz, 1H), 2.98 (d, *J* = 18.9 Hz, 1H), 2.57 - 2.37 (m, 6H), 2.33 (d, *J* = 14.8 Hz, 2H), 2.23 - 2.04 (m, 4H), 1.90 (td, *J* = 12.3, 3.7 Hz, 2H), 1.46 - 1.23 (m, 9H). ¹³C NMR (126 MHz, CDCl₃) δ 212.0, 210.0, 187.0, 186.7, 156.8, 156.5, 136.0, 134.7, 134.6, 134.0, 133.6, 133.5, 127.2, 127.1, 111.6, 110.9, 100.9, 100.3, 82.2, 77.4, 77.2, 76.9, 76.6, 72.0, 69.3, 68.3, 67.7, 65.2, 46.9, 35.0, 34.5, 33.7, 33.6, 29.8, 27.7, 25.0, 17.6, 17.2, 14.9. HRMS: [M + H]⁺ calculated for C38H45NO14 740.2918; found 740.2909.

### 7-[2,3-Dideoxy-4-ulo-α-L-fucopyranosyl-2-deoxy-α-L-fucopyranosyl-(1→4)- -2,3-dideoxy-3-dimethylamino-α-L-fucopyranoside]-idarubicinone (26)

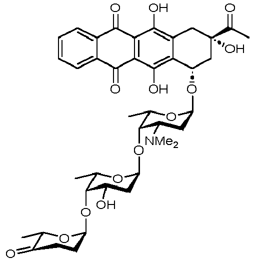

To a solution of **25** (20 mg, 27 umol) in EtOH (5.4 mL) and DCM (1.0 mL) was added 37% aq. CH₂O (0.20 mL) and the mixture was stirred for 40 minutes before addition of NaBH(OAc)₃ (11.2 mg, 52.8 umol, 1.95 eq). After stirring for 90 minutes, the mixture was poured into sat. aq. NaHCO₃ and extracted with DCM thrice. The organic layer was dried over Na₂SO₄ and concentrated *in vacuo.* Column chromatography on neutral silica gel (10:90 - 30:70 MeOH:DCM) gave the title compound as a red solid (12.4 mg, 16.1 umol, 60%). ¹H NMR (850 MHz, CDCl₃) δ 8.34 (dt, *J* = 5.9, 3.6 Hz, 2H), 7.91 - 7.78 (m, 2H), 5.61 - 5.50 (m, 1H), 5.28 (dd, *J* = 4.0, 2.1 Hz, 1H), 5.16 - 5.04 (m, 2H), 4.50 (dq, *J* = 10.3, 6.7 Hz, 2H), 4.17 - 4.10 (m, 1H), 4.02 (q, *J* = 6.5 Hz, 1H), 3.84 (d, *J =* 14.0 Hz, 1H), 3.70 (d, *J* = 2.8 Hz, 1H), 3.25 (dd, *J* = 18.7, 2.1 Hz, 1H), 3.00 (d, *J=* 18.6 Hz, 1H), 2.62 (s, 2H), 2.52 - 2.43 (m, 2H), 2.42 (s, 3H), 2.38 - 2.30 (m, 6H), 2.19 - 2.12 (m, 1H), 2.12 - 2.08 (m, 2H), 2.05 - 1.99 (m, 1H), 1.92 - 1.88 (m, 1H), 1.85 (td, *J* = 12.2, 3.7 Hz, 1H), 1.32 (d, *J* = 6.8 Hz, 3H), 1.30 (dd, *J* = 6.7, 3.2 Hz, 3H), 1.18 (d, *J* = 6.5 Hz, 3H). ¹³C NMR (214 MHz, CDCl₃) δ 211.8, 210.3, 187.0, 186.8, 173.5, 156.8, 156.6, 136.0, 134.7, 134.7, 134.0, 133.7, 133.5, 127.2, 127.1, 111.6, 110.9, 100.9, 100.2, 99.4, 82.6, 73.8, 73.7, 71.9, 69.3, 68.6, 67.2, 65.3, 61.8, 43.0, 35.0, 34.3, 33.7, 33.7, 32.1, 29.8, 28.7, 27.8, 25.5, 24.9, 18.2, 17.1, 14.9. HRMS: [M + H]⁺ calculated for C40H49NO14 768.3231; found 768.3221.

### Synthesis of additionalAnthracycline compounds 3-5, 6-14, 16 and 19-24

### Synthesis of compounds 3-5

The anthracycline analogues **3-5** were synthesized as described in Wander D. P. A., van der Zanden S. Y. et al. J Org Chem 2021, 86 (8), 5757-5770*,* and Wander D. P. A., van der Zanden S. Y. et al. J Med Chem 2020, 63 (21), 12814-12829*.*

### Synthesis of compounds 6-9

| 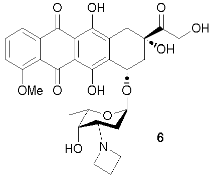 | 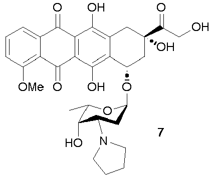 | 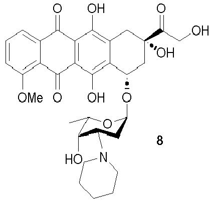 | 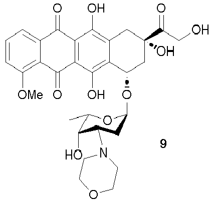 |
|---|---|---|---|
| Prepared according to General Procedure A from doxorubicin·HCl (50 mg) and diiodopropane to give the title compound as a red solid (17 mg, 29 pmol, 34%). | Prepared according to General Procedure A from doxorubicin · HCl (50 mg) and diiodopropane to give the title compound as a red solid (27 mg, 45 µmol, 53%). | Prepared according to General Procedure A from doxorubicin · HCl (100 mg) and diiodopentane to give the title compound as a red solid (81 mg, 0.13 mmol, 77%). | Prepared according to General Procedure A from doxorubicin · HCl (50 mg) and bis(2-iodo)ethyl ether to give the title compound as a red solid (32 mg, 52 pmol, 60%). |

### Synthesis of compound 10

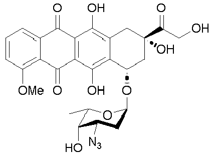

To a mixture of doxorubicin hydrochloride (200 mg, 0.340 mmol), potassium carbonate (72 mg, 0.51 mmol, 1.5 eq) and copper sulfate (cat. amount) in methanol (4 mL) was added imidazole-1-sulfonyl azide hydrochloride (22 mg, 0.1035 mmol, 3.3 eq) and the reaction mixture was stirred overnight. The mixture diluted with water and extracted with DCM thrice. The combined organic layers dried over Na₂SO₄ and concentrated *in vacuo.* Column chromatography (10:90 MeOH:DCM) gave 3'-azidodoxorubicin as a red solid (140 mg, 0.248 mmol, 72%).

### Synthesis of compound 11

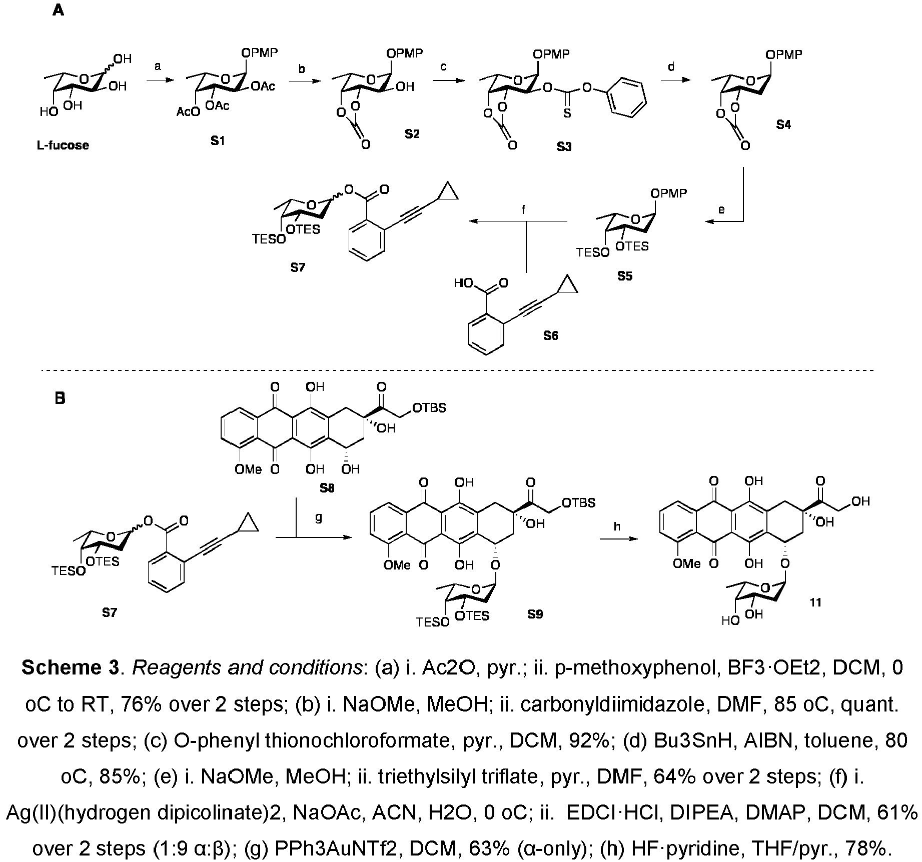

### Synthesis of compound 12

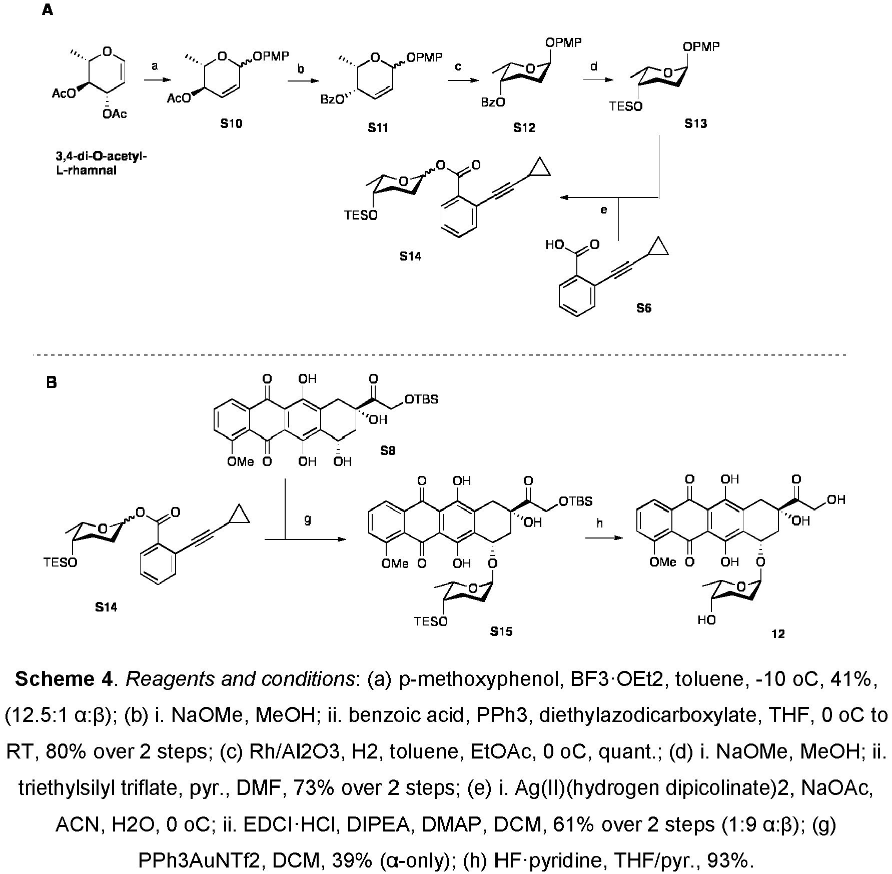

### Synthesis of compounds 13 & 14

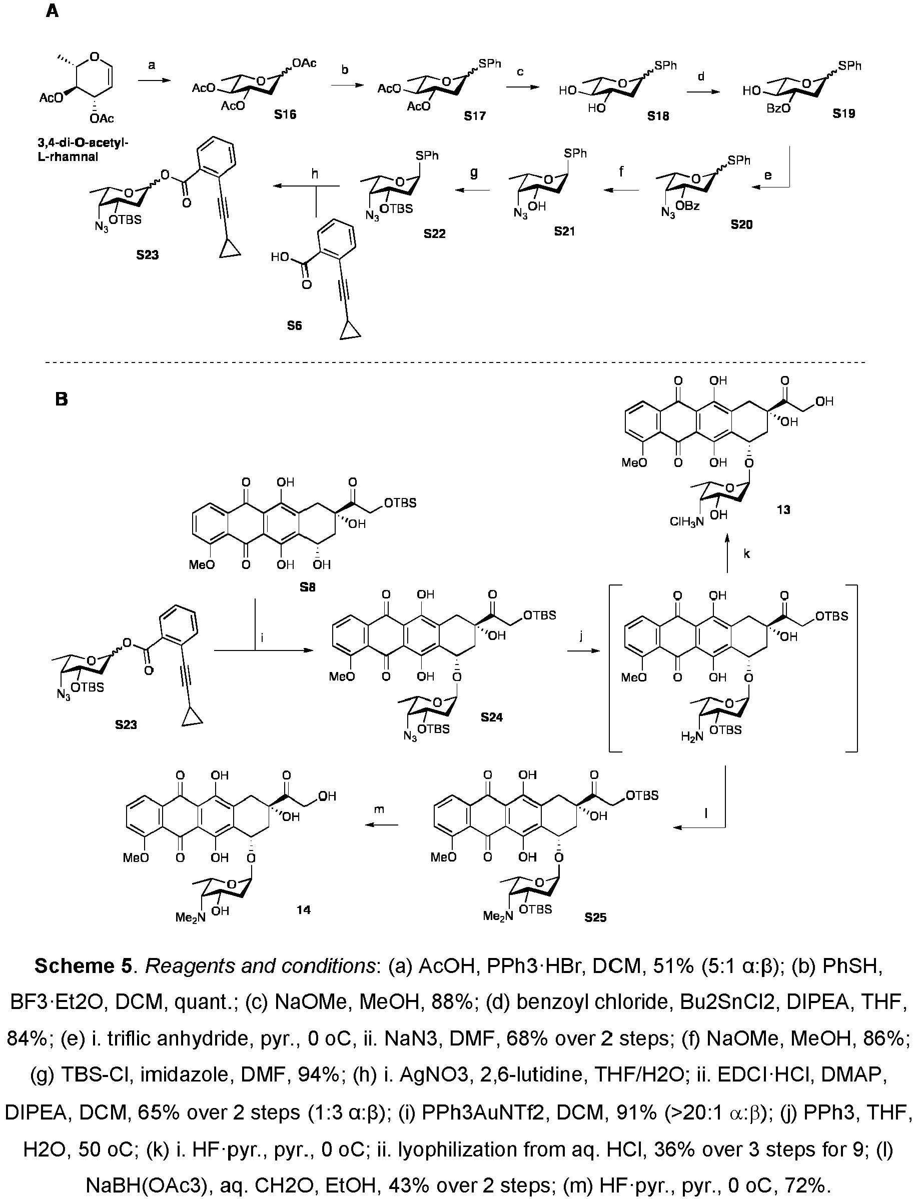

### Synthesis of compound 16

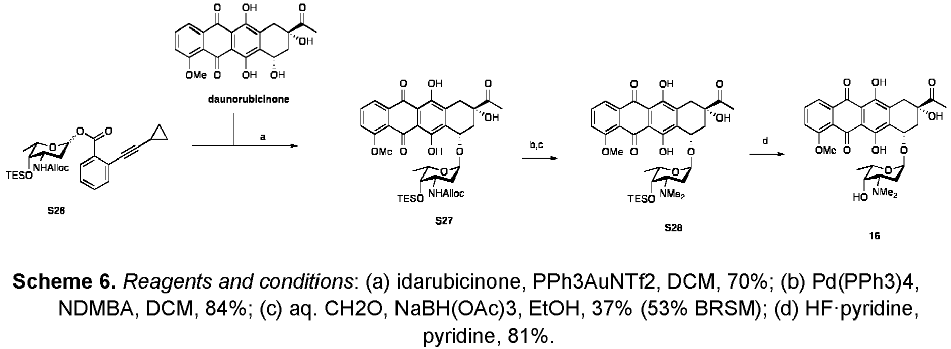

### Synthesis of compounds 19 & 20

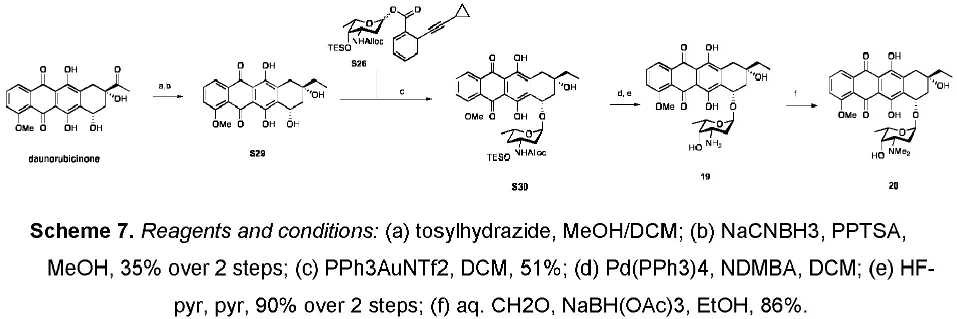

### Synthesis of compounds 21 & 22

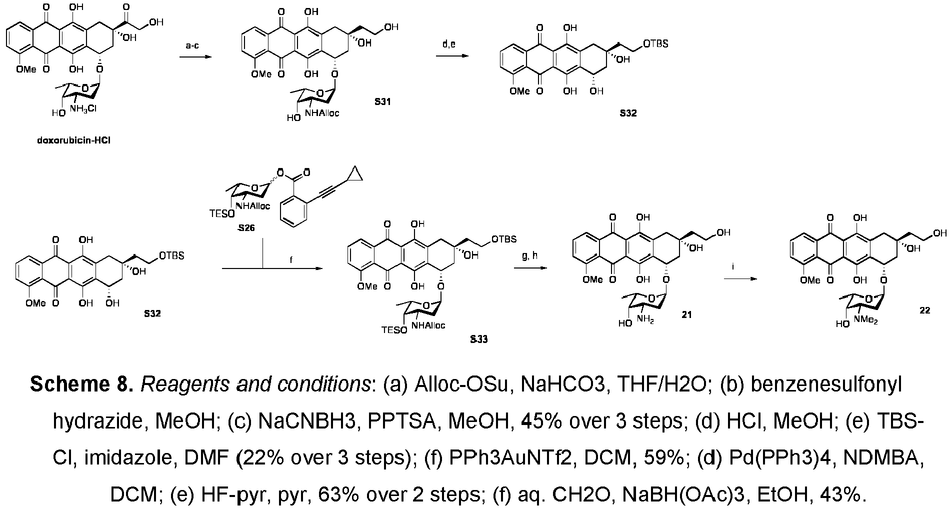

### Synthesis of compounds 23 & 24

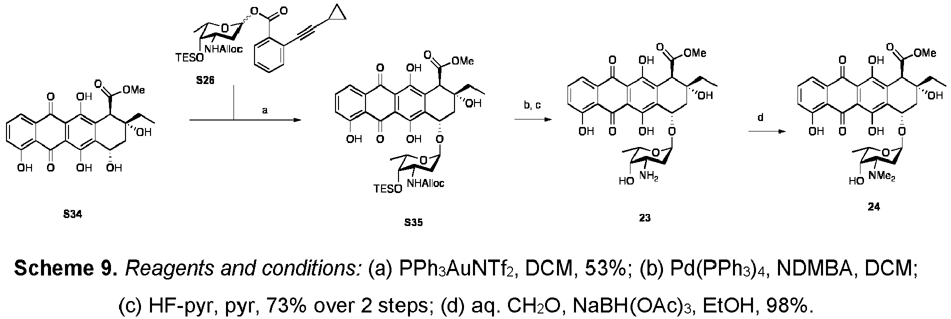

### Example 2: Assessment of compounds

### Cell cultures

K562 cells (B. Pang, Leiden University Medical Center, the Netherlands) were maintained in RPMI-1640 medium supplemented with 8% FCS. MelJuSo cells were maintained in IMDM supplemented with 8% FCS. MelJuSo cells stably expressing PAGFP-H2A were maintained in IMDM supplemented with 8% FCS and G-418, as described in Pang, B., Qiao, X., Janssen, L. et al. Nat Commun 4, 1908 (2013*).* U2Os cells (ATCC^{®} HTB-96) were maintained in DMEM medium supplemented with 8% FCS. Cell lines were maintained in a humidified atmosphere of 5% CO₂ at 37°C, regularly tested for the absence of mycoplasma and the origin of cell lines was validated using STR analysis.

### Quantification and statistical analysis

Each experiment was assayed in triplicate, unless stated otherwise. Error bars denote ±SD. Statistical analyses was performed using Prism 8 software (GraphPad Inc.): ns = not significant, *p = < 0.05, **p = < 0.01, ***p = < 0.001.

Experimental data having regard to compounds embodying the present invention and reference compounds are described below. The compounds were assessed for their potency to affect three biological processes:
i) The cytotoxicity in three relevant cancer cell lines (**assay 1**),
ii) DNA double-strand breaks (**assay 2**),
iii) Chromatin damage via histone eviction (**assay 3**).

**Assay 1.** Anthracyclines are often used in the treatment of acute myeloid leukaemia and other haematological malignancies. Therefore, the compounds shown in **FIG. 1** were assayed for their activity versus human myelogenous leukemic cell line K562 in order to determine their cytotoxicity *in vitro,* using the assay for short-term growth inhibition viability described herein. The data is recorded in **FIG. 2**A where the IC₅₀ values for all compounds are plotted. IC₅₀ is expressed in µM and represents the concentration of compound required to inhibit cell proliferation by 50%.

The data in **FIG. 2A** shows that compounds of the present inventions (**18 & 26**) can be considerably more cytotoxic than doxorubicin (**1**) which remains the foremost use clinical anthracycline variant. *N*,*N-*dimethylated-idarubicin trisaccharide (**26**) was the most effective compound tested; active at low nanomolar concentrations. With an IC₅₀ of 20 nM in K562 cells this variant is 16 times more cytotoxic than doxorubicin (**1**). The observed cytotoxicity of compounds embodying the present invention appeared consistent across cell types since similar cytotoxicity profiles were observed in cell lines from different cancer origins (**FIG. 2B**).

**Assay 3.** In order to assess the mechanism of action of the compounds embodying the present invention, the compounds were also assayed to evaluate their DNA damaging activity. This was determined by assessing γH2AX levels (see Kuo, L. J., Yang, L. X. In Vivo. April 2008, pp 305-310*)* using the assay for Western blot analysis described herein. Etoposide (a podophyllotoxin based topoisomerase II inhibitor) was included as positive control for DNA break formation. The results are set out in **FIGS. 3A & 3B** and show that compounds of the present invention (**18 & 26**) did not induce DNA damage. Indeed, most compounds/variants with a tertiary amine on the sugar attached to the tetracyclic aglycon did not induce DNA damage.

**Assay 2.** Because anthracycline drugs can also evict histones, including H2AX, the levels of phosphorylated H2AX might be affected. To determine DNA double-strand break induction by the compounds embodying the present invention at the DNA level, we assessed the DNA damage capacity of all the compounds by using constant field gel electrophoresis (CFGE), as described below, a more direct method to detect DNA breaks. The results are set out in **FIGS. 3C & 3D**. This complementary assay to study DNA damage confirmed the γH2AX observations showing that compounds embodying the present invention (**18 & 26**) do not induce DNA.

**Assay 4.** This assay visualised histone eviction and subsequently, the chromatin damage induced by the compounds embodying the present invention could be assessed. The method followed is described in the assay for microscopy analysis described herein. For all tested derivatives the rate of histone eviction (EC₅₀, the time at which 50% of the initial signal is reduced) was recorded and the results are plotted in **FIG. 4A****.** Compounds embodying the present invention (**18 & 26**) all displayed superior chromatin damage activity relative to doxorubicin (**1**). Compound **26** of the present invention displayed the fastest histone eviction activity and outperformed both aclarubicin (**2**) and idarubicin (**17**) from which its hybrid structure is derived.

**FIG 5****.** which plots cytotoxicity IC₅₀ against histone eviction EC₅₀ shows a strong correlation between the two, supporting the hypothesis that improved chromatin damage leads to superior cytotoxicity.

**FIG. 6** plots cytotoxicity IC₅₀ against DNA damage and shows a general lack of correlation between anthracyclines which induce DNA double-strand breaks and their cytotoxicity profile.

The **assays 1-4** show that compound **26** which forms an embodiment of the present invention is more toxic than doxorubicin (**1**), aclarubicin (**2**) and idarubicin (**17**), more efficient in terms of histone eviction, whilst not inducing any DNA double-strand breaks. Compound **18** which forms an embodiment of the present invention has improved or comparable toxicity and rate of histone eviction relative to doxorubicin **(1)** and idarubicin **(17),** however unlike both compounds **1** and **17,** compound **18** does not induce any DNA double-strand breaks.

## Claims

1. A compound of formula I: 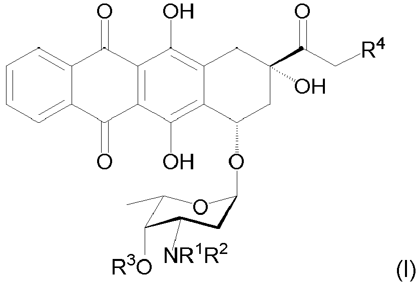 wherein:
R¹ is a substituted or unsubstituted C₁₋₄ alkyl;
R² is a substituted or unsubstituted C₁₋₄ alkyl;
R³ is selected from H, monosaccharide, and disaccharide;
R⁴ is selected from H, and OH,
or a pharmaceutically acceptable salt, stereoisomer, solvate, or prodrug thereof.

2. The compound of claim 1, selected from: 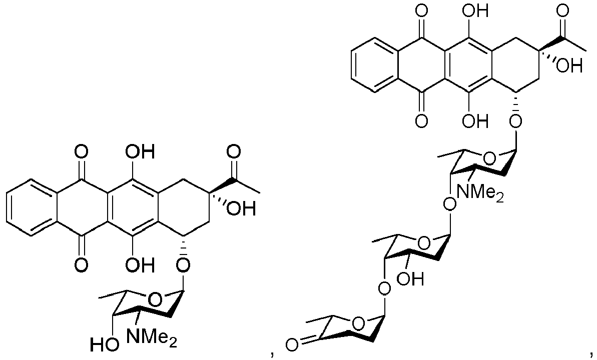 or a pharmaceutically acceptable salt, stereoisomer, solvate, or prodrug thereof.

3. The compound of claim 1, wherein R¹ is selected from CH₃ and CH₂CH₃.

4. The compound of claim 1 or claim 3, wherein R² is selected from CH₃ and CH₂CH₃.

5. The compound of any of claims 1, 3 or 4, wherein R¹ is CH₃ and R² is CH₃.

6. The compound of any of claims 1 or 3 to 5, wherein R³ is selected from H and 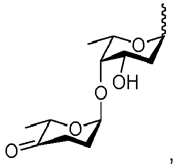 where 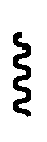 designates the point of attachment to the rest of the compound.

7. The compound of any of claims 1 or 3 to 6, wherein R⁴ is H.

8. The compound of any preceding claim, wherein the compound has an IC₅₀ of less than about 100 nM against cell line K562 and wherein the fraction of broken DNA damage in the K562 cells is less than about 0.10.

9. The compound of any preceding claim, wherein the compound has an IC₅₀ of less than about 150 nM against cell line MelJuSo and/or an IC₅₀ of less than about 150 nM against cell line U2Os.

10. A formulation, comprising the compound of any preceding claim and optionally a pharmaceutically acceptable carrier.

11. The formulation of claim 10, wherein the formulation is an oral formulation.

12. The compound of any of claims 1 to 9, or formulation of claim 10 or claim 11, for use as a medicament.

13. The compound of any of claims 1 to 9, or formulation of claim 10 or claim 11, for use in the treatment of cancer.

14. The compound or formulation for use of claim 13, wherein the cancer is selected from a haematological cancer or a solid tumor, optionally wherein the cancer is a leukaemia (such as acute myeloid leukaemia (AML) or acute lymphatic leukaemia (ALL)), lymphoma, or breast cancer.

15. The compound or formulation for use of claim 13 or claim 14, wherein the cancer comprises ATP binding cassette B1 (ABCB1) OE chemotherapy resistance mechanism.
